# EUROPEAN PATENT SPECIFICATION

(11) **EP 1 996 240 B1**
(45) Date of publication and mention of the grant of the patent: **18.07.2012**
(21) Application number: 06745240.9
(22) Date of filing: 21.03.2006
(51) Int. Cl.: A61L 2/07, A61L 2/06, A61C 19/00, B05B 1/26

(54) **STEAM DELIVERY DEVICE**
DAMPFABGABEVORRICHTUNG
APPAREIL D'ÉMISSION DE VAPEUR

(43) Date of publication of application: 03.12.2008
(73) Proprietor: POLTI S.p.A., 22070 Bulgarograsso (Como) (IT)
(72) Inventor: POLTI, Franco, I-22070 Olgiate Comasco CO (IT); CAPPI, Stefano, I-22100 Como (IT); DAMIANO, Peter, I-87050 Mangone CS (IT)
(74) Representative: Cicogna, Franco
(86) International application number: PCT/IT2006/000174
(87) International publication number: WO 2007/108021

(56) References cited:
- WO-A-99/38539
- DE-C1- 19 811 587
- US-A1- 2004 050 877

## Description

The present invention relates to a steam delivery device comprising a nozzle from the orifice of which the steam supplied from a pressurised steam source is emitted.

It is known that steam, which may be superheated, is used widely in all those applications which require thorough disinfection and sanitization of the environment such as, for example, train interiors, cinema halls, theatres, but also and in particular the furnishings, instruments and apparatus present in medical and veterinary clinics, dentists studios, surgical centres, ambulances, operating theatres and similar environments.

Document DE 198 11 587 C1 discloses a steam delivery device for the disinfection of surfaces which comprises a nozzle from the orifice of which the steam supplied from a pressurized steam source is emitted, the device being provided with a tubular chamber positioned around and extending downstream of the outlet orifice of the nozzle. The tubular chamber is closed at the nozzle side and open at the opposite side through which side the steam is delivered into the environment towards the objects to be sterilized. The cross-sectional area of the tubular chamber extending normal to its axis is greater than the cross-sectional area of the orifice of the nozzle, said tubular chamber extending concentrically with the longitudinal axis of the nozzle over a predetermined straight section.

In the dental environment, in particular, in order to deal with hygiene problems, normally costly autoclave sterilization and instrument washing equipment is used, said equipment, although being very effective, having a complex operating procedure and long process times.

This equipment is normally used once a day or after the treatment of patients with viral infections.

The set of furnishings, instruments and apparatus normally used are instead washed and sanitized by the surgery assistant, using alcohol-based disinfectants which are atomized by means of sprayers, and dried with paper towels.

This operation is indispensable after treating each patient and requires long operating times.

Systems using saturated or also superheated steam have been proposed, the latter being sprayed against the objects to be sterilized by means of a high-pressure delivery device.

However, these systems which use steam, while they are very effective for cleaning domestic or industrial environments, instead have major limitations when used for sterilization applications of the type indicated above.

In fact, in the known delivery devices, the jet of steam, no matter how powerful it is in terms of pressure and despite being supplied by the source at high temperatures, when it strikes the surfaces of the objects to be sterilized, has temperatures which are no longer suitable for the purpose, i.e. for the destruction of the microbial activity present on the said objects.

The object of the present invention is to provide a steam delivery device which allows the disinfection and sanitization of surgical and/or medical instruments and equipment, in particular but not exclusively those which are used in a dentist's studio, rapidly and in a very effective manner as regards the particular microbial activity which is to be destroyed.

The object is achieved with a steam delivery device in accordance with Claim 1 and claim 6.

The invention will now be described in greater detail with reference to an example of practical embodiment, provided solely by way of a non-limiting example, illustrated in the accompanying drawings in which: .
- Figure 1 shows a vertically sectioned view of the steam delivery device according to the invention;
- Figure 2 shows the horizontally sectioned view of the delivery device along the line II-II of Figure 1.

With reference to the abovementioned figures, 1 schematically indicates a conventional steam source, the capacity of which to produce steam may be varied according to requirements. The source 1 may be, by way of example, a boiler with a 1500 W capacity supplying steam at a pressure of 3 bar or a boiler with a much higher capacity supplying steam at a pressure of 10 bar.

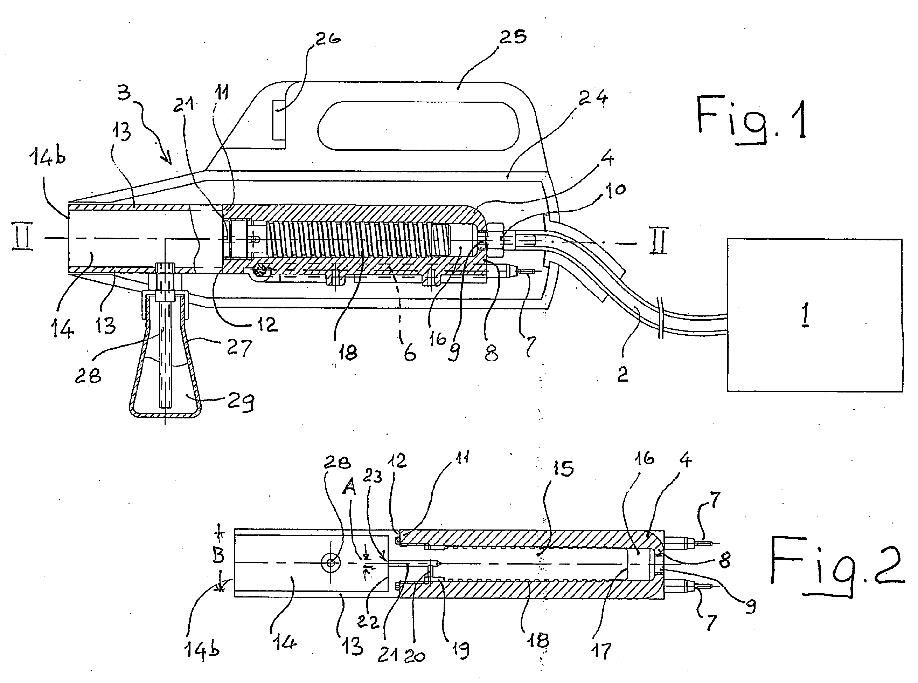

The steam supplied from the source 1, for example a boiler, is conveyed, by means of a pipe 2, which is for example flexible, and also conventional, to the delivery device according to the invention which is denoted overall by 3 in Figure 1.

The latter comprises a tubular body 4 having an axial cavity formed inside it. The body 4 is provided with a heating device, for example a conventional electric resistance 6 equipped with terminals 7 for the powering thereof.

At one of its ends the tubular body 4 is closed by the end wall 8 which is provided with an opening 9 to which the steam supply pipe 2 is connected by means of a union 10, shown in Figure 1.

The opposite end 11 of the tubular body 4 is open and intended to be sealingly closed by the annular shoulder 12 of a part 13 arranged coaxially with the body 4.

This part 13 has a tubular chamber 14, preferably with a circular cross-section, which extends axially and concentrically with respect to the body 4 on one side, relative to the annular shoulder 12, and a cylindrical body 15 which extends on the opposite side, being housed inside the axial cavity 5 of the body 4 with the formation of a chamber 16 between its end 17 and the end wall 8.

The surface of the cylindrical body 15 has, formed therein, a helical groove which, with the internal cylindrical surface of the body 4, defines a helical duct 18. The latter is connected, on one side, to the chamber 16 into which the steam is supplied from the pipe 2 and, on the other side, to an annular groove 19 formed in the cylindrical body 15.

This annular groove 19 is in fluid communication with a radial duct 20 formed in the body 15 and this radial duct 20 is, in turn, in communication with a duct 21 formed axially inside the body 15, closed at the bottom end and open via an orifice 23 emerging inside the space defined by the tubular chamber 14.

The latter is therefore closed at the bottom 22 where the orifice 23 is formed and freely open with an opening 14b on the opposite side.

In accordance with the invention, the cross-sectional area "B" of the chamber 14 and its opening 14b towards the outside is greater than the cross-sectional area "A" of the orifice 23 of the duct 21 by a factor ranging between 30 and 150.

The assembly formed by the body 4 and the part 13 may be housed in a fixed location or inside a casing 24 with handle 25 for manoeuvring it, as shown in Figure 1. The handle 25 may also have, arranged thereon, a pushbutton 26 for activating and interrupting the supply of steam from the source 1, in a conventional manner.

In accordance with the invention, the delivery device 3 may comprise a tank 27 connected radially to the part 13 and in communication with the chamber 14 by means of a pipe 28 which draws from the bottom of the tank.

The latter is intended to contain a liquid, denoted by 29 in Figure 1, to be added to the steam during delivery thereof. The liquid is a disinfectant or a cleansing agent or an aromatic substance and suction thereof inside the chamber 14 may take place, for example, by means of the Venturi effect along the pipe 28, caused by the flow of steam being delivered.

From the above description it is clear that, following operation of the pushbutton 26 or an equivalent control device, if the apparatus is in a fixed location, the steam is supplied from the source 1 to the chamber 16 of the body 4 from where, via the helical duct 18, the groove 19 and radial duct 20, it reaches the axial duct 21 which performs the action of a delivery nozzle.

During travel along the helical duct 18, if the electric resistance 6 is activated, the steam supplied from the source 1, if required, is superheated or in any case rendered totally dry.

The steam, therefore, in the region of the orifice 23 emerges inside the space defined by the tubular chamber 14 which forms a restricted space relative to the surrounding free environment. The expansion of the steam inside the restricted environment of the chamber 14 therefore takes place with a cooling action which is notably less than that which would occur if the orifice 23 were to emerge directly inside the external free environment.

It follows that, at the outlet of the tubular section of the chamber 14, the steam still possesses a high temperature which remains substantially unvaried until it strikes the tools or the equipment to be sterilized.

The efficiency of the delivery device according to the present invention was determined by means of a series of experimental operating tests using steam supplied from a boiler at a pressure of 3.7 bar with a throughput of 100 grammes/minute, without the use of a superheater. The temperature of the steam measured at the orifice 23 was, in all the tests, kept at 100°C.

The tests were carried out detecting the temperature at which the steam, emitted from the delivery device according to the invention, managed to strike the surface to be sterilized situated at 30 mm from the opening 14b of the chamber 14 upon variation in the cross-sectional area "A" of the orifice 23 and variation in the cross-sectional area "B" of the opening 14b. The axial length of the chamber 14 was, in all the tests, equal to 120 mm.

The results of the experimental tests referred to above are shown in the following table:

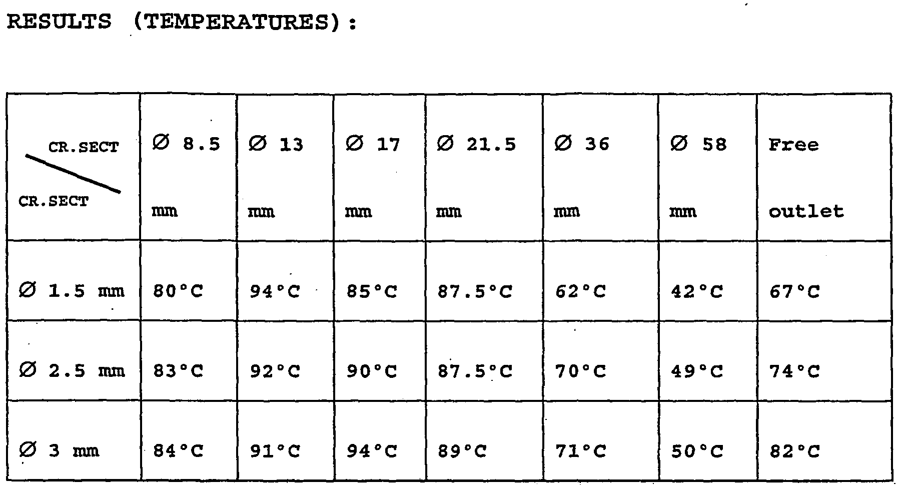

As can be seen from the data shown in the Table, the maximum efficiency, in terms of temperature still possessed by the jet of steam which leaves the delivery device according to the invention, is obtained with an orifice 23 having a diameter of 1.5 mm and with an opening 14b, of the tubular chamber 14, having a diameter of 13 mm or with an orifice having a diameter of 3 mm together with an opening 14b of the tubular chamber having a diameter of 17 mm. In both cases, the temperature of the steam measured on the surface of the object to be sterilized was equal to 94°C.

However, the efficiency in terms of temperature and therefore sterilization was acceptable also with combinations of different diameters whenever the cross-sectional area "B" of the opening 14b had a value between 30 and 150 times the cross-sectional area "A" of the orifice 23, for the same axial length of the tubular chamber 14.

The dimensions and the form of the casing 24 and the materials used for manufacture of the delivery device may obviously vary depending on the requirements, without thereby departing from the scope of the invention as described above and claimed below.

## Claims

1. A sterilizing steam delivery device (1) for delivering a sterilizing steam flow having a steam delivery pressure from substantially 3 to 10 bar and a steam delivery temperature up to 94°C for sterilizing objects, said device comprising a tubular body (4) having first (17) and second (11) tubular body ends, said first end being closed by an end wall (8) including an opening (9) to which a steam supply pipe (2) is connected, said second end (11) of said tubular body being open and adapted to be tightly closed by an annular shoulder (12) of a part (13) arranged coaxially with said tubular body (4), said part (13) having a tubular chamber (14) extending axially and concentrically with respect to said tubular body (4) on one side, relative to said annular shoulder (12), and a cylindrical body (15) extending on an opposite side being housed in said tubular body (4) thereby forming a further chamber (16) between the first end (17) of said cylindrical body (4) and the end wall (8), said cylindrical body (15) having a helical groove defining, with an internal cylindrical surface of said tubular body, a helical duct (18) connected, on one side, to said further chamber (16) into which said steam is supplied from said steam supply pipe (2) and, on another side, to an annular groove (19) formed in said cylindrical body (15) and fluidly communicating with a radial duct (20) formed in said cylindrical body (15) and in turn communicating with a duct nozzle (21) formed axially in said cylindrical body (15) and being closed at a bottom end and including a nozzle orifice (23) communicating with said tubular chamber, said tubular chamber (14) being otherwise closed, at a bottom thereof where said nozzle orifice (23) is formed and freely open at an opposite opening of said tubular chamber, the tubular chamber being positioned around the zone where the orifice emerges, said tubular chamber extending concentrically with the longitudinal axis of the nozzle over a predetermined straight section thereby the steam emerging from the nozzle orifice is urged to stream inside the tubular chamber, said tubular chamber (14) having a cross-sectional area greater than a cross-sectional area of the nozzle orifice (23) by a factor ranging between 30 and 150 , said tubular body (4) further including an on/off controlled electric resistance steam drying and superheating device (6), and wherein said steam delivery device further comprises a disinfectant or cleansing or aromatic liquid tank (27) radially connected to said part (13) and communicating with said tubular chamber (14) by a bottom liquid drawing pipe (28), for adding to said steam being delivered said liquid by a Venturi effect caused by the delivered steam flow.

2. A sterilizing steam delivery device according to claim 1, wherein said cross-section of said tubular chamber (14) is circular.

3. A sterilizing steam delivery device according to claim 1, including a casing (24) with an operating handle (25) fixed to said tubular chamber (14), said operating handle (25) comprising a control device for on/off controlling a delivery of said steam toward said nozzle.

4. A sterilizing steam delivery device according to claim 1, wherein said nozzle orifice (23) has a diameter of 1.5 mm and said opening of said tubular chamber (14) has a diameter of 13 mm.

5. A sterilizing steam delivery device according to claim 1, wherein said nozzle orifice (23) has a diameter of 3 mm and said opening of said tubular chamber (14) has a diameter of 17 mm.

6. A sterilizing steam delivery device (1) for delivering a sterilizing steam flow having a steam delivery pressure from 3 to 10 bar and a steam delivery temperature up to 94°C for sterilizing dental implements, said device comprising a tubular body (4) having first (17) and second (11) tubular body ends, said first end being closed by an end wall (8) including an opening (9) to which a steam supply pipe (2) is connected, said second end (11) of said tubular body being open and adapted to be tightly closed by an annular shoulder (12) of a part (13) arranged coaxially with said tubular body (4), said part (13) having a tubular chamber (14) extending axially and concentrically with respect to said tubular body (4) on one side, relative to said annular shoulder (12), and a cylindrical body (15) extending on an opposite side being housed in said tubular body (4) thereby forming a further chamber (16) between the first end (17) of said cylindrical body (4) and the end wall (8), said cylindrical body (15) having a helical groove defining, with an internal cylindrical surface of said tubular body, a helical duct (18) connected, on one side, to said further chamber (16) into which said steam is supplied from said steam supply pipe (2) and, on another side, to an annular groove (19) formed in said cylindrical body (15) and fluidly communicating with a radial duct (20) formed in said cylindrical body (15) and in turn communicating with a duct nozzle (21) formed axially in said cylindrical body (15) and being closed at a bottom end and including a nozzle orifice (23) communicating with said tubular chamber, said tubular chamber (14) being otherwise closed, at a bottom thereof where said nozzle orifice (23) is formed and freely open at an opposite opening of said tubular chamber, the tubular chamber being positioned around the zone where the orifice emerges, said tubular chamber extending concentrically with the longitudinal axis of the nozzle over a predetermined straight section thereby the steam emerging from the nozzle orifice is urged to stream inside the tubular chamber, said tubular chamber (14) having a cross-sectional area greater than a cross-sectional area of the nozzle orifice (23) by a factor ranging between 30 and 150, said tubular body (4) further including an on/off controlled electric resistance steam drying and superheating device (6), and wherein said steam delivery device further comprises a disinfectant or cleansing or aromatic liquid tank (27) radially connected to said part (13) and communicating with said tubular chamber (14) by a bottom liquid drawing pipe (28), for adding to said steam being delivered said liquid by a Venturi effect caused by the delivered steam flow.

## Patentansprüche

1. Sterilisierdampfabgabevorrichtung (1) zum Abgeben eines Sterilisierdampfstroms mit einem Dampfabgabedruck von im Wesentlichen 3 bis 10 Bar und einer Dampfabgabetemperatur von bis zu 94 °C zum Sterilisieren von Objekten, die Vorrichtung umfassend einen Röhrenkörper (4) mit einem ersten (17) und zweiten (11) Röhrenkörperende, wobei das erste Ende durch eine Endwand (8) mit einer Öffnung (9), an die ein Dampfabgaberohr (2) angeschlossen ist, geschlossen ist, wobei das zweite Ende (11) des Röhrenkörpers offen ist und dazu geeignet ist, durch einen ringförmigen Ansatz (12) eines Teils (13), das koaxial mit dem Röhrenkörper (4) angeordnet ist, dicht geschlossen zu sein, wobei das Teil (13) eine Röhrenkammer (14) aufweist, die axial und konzentrisch bezüglich des Röhrenkörpers (4) auf einer Seite, bezüglich des ringförmigen Ansatzes (12) und eines zylindrischen Körpers (15) verläuft, der auf einer gegenüberliegenden Seite verläuft und in dem Röhrenkörper (4) untergebracht ist, wodurch eine weitere Kammer (16) zwischen dem ersten Ende (17) des zylindrischen Körpers (4) und der Endwand (8) ausgebildet ist, wobei der zylindrische Körper (15) eine spiralförmige Nut aufweist, die mit einer zylindrischen Innenfläche des Röhrenkörpers einen spiralförmigen Durchgang (18) definiert, der auf einer Seite mit der weiteren Kammer (16), in die der Dampf von dem Dampfabgaberohr (2) abgegeben wird, und auf einer anderen Seite mit einer ringförmigen Nut (19) verbunden ist, die in dem zylindrischen Körper (15) ausgebildet ist und in Fluidverbindung mit einem radialen Durchgang (20) steht, der in dem zylindrischen Körper (15) ausgebildet ist und wiederum mit einer Durchgangsdüse (21) in Verbindung steht, welche axial in dem zylindrischen Körper (15) ausgebildet ist und am unteren Ende geschlossen ist und eine Düsenöffnung (23) enthält, die mit der Röhrenkammer in Verbindung steht, wobei die Röhrenkammer (14) andererseits an einer Unterseite davon, wo die Düsenöffnung (23) ausgebildet ist, geschlossen ist und an einer gegenüberliegenden Öffnung der Röhrenkammer unbehindert offen ist, wobei die Röhrenkammer um den Bereich herum angeordnet ist, wo die Öffnung austritt, wobei die Röhrenkammer konzentrisch mit der Längsachse der Düse über einen vorgegebenen geraden Teilabschnitt verläuft, wodurch der Dampf, der aus der Düsenöffnung austritt, zum Strömen innerhalb der Röhrenkammer gezwungen ist, wobei die Röhrenkammer (14) eine Querschnittsfläche aufweist, die um einen Faktor im Bereich zwischen 30 und 150 größer als eine Querschnittsfläche der Düsenöffnung (23) ist, wobei der Röhrenkörper (4) ferner eine zweipunktgeregelte Elektrowiderstandsdampftrocknungs- und - überhitzungsvorrichtung (6) enthält, und wobei die Dampfabgabevorrichtung ferner einen Desinfektionsmittel- oder Spülungs- oder Aromatikflüssigkeitstank (27), der radial an das Teil (13) angeschlossen ist und mit der Röhrenkammer (14) durch ein unteres Flüssigkeitsabzugsrohr (28) in Verbindung steht, zum Zusetzen der Flüssigkeit durch einen Venturi-Effekt, der durch den zugeführten Dampfstrom erzeugt ist, zu dem Dampf, der abgegeben wird, umfasst.

2. Sterilisierdampfabgabevorrichtung nach Anspruch 1, wobei der Querschnitt der Röhrenkammer (14) kreisförmig ist.

3. Sterilisierdampfabgabevorrichtung nach Anspruch 1, enthaltend ein Gehäuse (24) mit einem Betätigungsgriff (25), das an der Röhrenkammer (14) befestigt ist, der Betätigungsgriff (25) umfassend eine Regelvorrichtung zum Zweipunktregeln einer Abgabe des Dampfs zur Düse hin.

4. Sterilisierdampfabgabevorrichtung nach Anspruch 1, wobei die Düsenöffnung (23) einen Durchmesser von 1,5 mm aufweist und die Öffnung der Röhrenkammer (14) einen Durchmesser von 13 mm aufweist.

5. Sterilisierdampfabgabevorrichtung nach Anspruch 1, wobei die Düsenöffnung (23) einen Durchmesser von 3 mm aufweist und die Öffnung der Röhrenkammer (14) einen Durchmesser von 17 mm aufweist.

6. Sterilisierdampfabgabevorrichtung (1) zum Abgeben eines Sterilisierdampfstroms mit einem Dampfabgabedruck von im Wesentlichen 3 bis 10 Bar und einer Dampfabgabetemperatur von bis zu 94 °C zum Sterilisieren von Dentalinstrumenten, die Vorrichtung umfassend einen Röhrenkörper (4) mit einem ersten (17) und zweiten (11) Röhrenkörperende, wobei das erste Ende durch eine Endwand (8) geschlossen ist, die eine Öffnung (9) enthält, an die ein Dampfabgaberohr (2) angeschlossen ist, wobei das zweite Ende (11) des Röhrenkörpers offen ist und dazu geeignet ist, durch einen ringförmigen Ansatz (12) eines Teils (13), das koaxial mit dem Röhrenkörper (4) angeordnet ist, dicht geschlossen zu sein, wobei das Teil (13) eine Röhrenkammer (14) aufweist, die axial und konzentrisch bezüglich des Röhrenkörpers (4) auf einer Seite, bezüglich des ringförmigen Ansatzes (12) und eines zylindrischen Körpers (15) verläuft, der auf einer gegenüberliegenden Seite verläuft und in dem Röhrenkörper (4) untergebracht ist, wodurch eine weitere Kammer (16) zwischen dem ersten Ende (17) des zylindrischen Körpers (4) und der Endwand (8) ausgebildet ist, wobei der zylindrische Körper (15) eine spiralförmige Nut aufweist, die mit einer zylindrischen Innenfläche des Röhrenkörpers einen spiralförmigen Durchgang (18) definiert, der auf einer Seite mit der weiteren Kammer (16), in die der Dampf von dem Dampfabgaberohr (2) abgegeben wird, und auf einer anderen Seite mit einer ringförmigen Nut (19) verbunden ist, die in dem zylindrischen Körper (15) ausgebildet ist und in Fluidverbindung mit einem radialen Durchgang (20) steht, der in dem zylindrischen Körper (15) ausgebildet ist und wiederum mit einer Durchgangsdüse (21) in Verbindung steht, welche axial in dem zylindrischen Körper (15) ausgebildet ist und am unteren Ende geschlossen ist und eine Düsenöffnung (23) enthält, die mit der Röhrenkammer in Verbindung steht, wobei die Röhrenkammer (14) andererseits an einer Unterseite davon, wo die Düsenöffnung (23) ausgebildet ist, geschlossen ist und an einer gegenüberliegenden Öffnung der Röhrenkammer unbehindert offen ist, wobei die Röhrenkammer um den Bereich herum angeordnet ist, wo die Öffnung austritt, wobei die Röhrenkammer konzentrisch mit der Längsachse der Düse über einen vorgegebenen geraden Teilabschnitt verläuft, wodurch der Dampf, der aus der Düsenöffnung austritt, zum Strömen innerhalb der Röhrenkammer gezwungen ist, wobei die Röhrenkammer (14) eine Querschnittsfläche aufweist, die um einen Faktor im Bereich zwischen 30 und 150 größer als eine Querschnittsfläche der Düsenöffnung (23) ist, wobei der Röhrenkörper (4) ferner eine zweipunktgeregelte Elektrowiderstandsdampftrocknungs- und - überhitzungsvorrichtung (6) enthält, und wobei die Dampfabgabevorrichtung ferner einen Desinfektionsmittel- oder Spülungs- oder Aromatikflüssigkeitstank (27), der radial an das Teil (13) angeschlossen ist und mit der Röhrenkammer (14) durch ein unteres Flüssigkeitsabzugsrohr (28) in Verbindung steht, zum Zusetzen der Flüssigkeit durch einen Venturi-Effekt, der durch den zugeführten Dampfstrom erzeugt ist, zu dem Dampf, der abgegeben wird, umfasst.

## Revendications

1. Appareil d'émission de vapeur de stérilisation (1) permettant d'émettre un flux de vapeur de stérilisation ayant une pression de sortie de la vapeur sensiblement comprise entre 3 et 10 bars et une température de sortie de la vapeur pouvant atteindre 94 °C dans le but de stériliser des objets, ledit appareil comprenant un corps tubulaire (4) ayant une première (17) et une seconde (11) extrémités de corps tubulaire, ladite première extrémité étant fermée par une paroi d'extrémité (8) incluant une ouverture (9) à laquelle un tuyau d'alimentation en vapeur (2) est raccordé, ladite seconde extrémité (11) dudit corps tubulaire étant ouverte et conçue pour être fermée hermétiquement par un épaulement annulaire (12) d'une partie (13) agencée coaxialement avec ledit corps tubulaire (4), ladite partie (13) ayant une chambre tubulaire (14) qui s'étend axialement et concentriquement par rapport au dit corps tubulaire (4) d'un côté, par rapport au dit épaulement annulaire (12), et un corps cylindrique (15) qui s'étend sur un côté opposé étant logé dans ledit corps tubulaire (4) en formant ainsi une autre chambre (16) entre la première extrémité (17) dudit corps cylindrique (4), et la paroi d'extrémité (8), ledit corps cylindrique (15) présentant une rainure hélicoïdale définissant, avec une surface cylindrique interne dudit corps tubulaire, une conduite hélicoïdale (18) raccordée, d'un côté, à ladite autre chambre (16), dans laquelle ladite vapeur est alimentée, en provenance dudit tuyau d'alimentation en vapeur (2) et, sur l'autre côté, à une rainure annulaire (19) formée dans ledit corps cylindrique (15) et en communication fluidique avec une conduite radiale (20) formée dans ledit corps cylindrique (15) et communicant à son tour avec une buse de conduite (21) formée axialement dans ledit corps cylindrique (15) et étant fermée au niveau d'une extrémité inférieure et incluant un orifice de buse (23) communiquant avec ladite chambre tubulaire, ladite chambre tubulaire (14) étant par ailleurs fermée, au niveau du fond de celle-ci, à l'endroit où ledit orifice de buse (23) est formé et ouverte librement au niveau d'une ouverture opposée de ladite chambre tubulaire, ladite chambre tubulaire étant positionnée autour de la zone dans laquelle l'orifice émerge, ladite chambre tubulaire s'étendant concentriquement avec l'axe longitudinal de la buse sur une section droite prédéterminée, grâce à quoi la vapeur émergeant de l'orifice de la buse est poussée à s'écouler à l'intérieur de la chambre tubulaire, ladite chambre tubulaire (14) ayant une section transversale supérieure à une section transversale de l'orifice de la buse (23) d'un facteur compris entre 30 et 150, ledit corps tubulaire (4) incluant en outre un appareil de séchage et de surchauffe de la vapeur à commande de marche-arrêt par une résistance électrique (6), et dans lequel ledit appareil d'émission de vapeur comprend en outre un réservoir à liquide désinfectant ou nettoyant ou aromatique (27) raccordé radialement à ladite partie (13) et communiquant avec ladite chambre tubulaire (14) grâce à un tuyau de vidange de liquide en fond (28), afin d'ajouter à ladite vapeur émise ledit liquide grâce à un effet Venturi provoqué par le flux de vapeur émis.

2. Appareil d'émission de vapeur de stérilisation selon la revendication 1, dans lequel ladite section transversale de ladite chambre tubulaire (14) est circulaire.

3. Appareil d'émission de vapeur de stérilisation selon la revendication 1, incluant un boîtier (24) muni d'une poignée de fonctionnement (25) fixée à ladite chambre tubulaire (14), ladite poignée de fonctionnement (25) comprenant un appareil de commande pour la commande marche-arrêt de l'émission de ladite vapeur vers ladite buse.

4. Appareil d'émission de vapeur de stérilisation selon la revendication 1, dans lequel ledit orifice de buse (23) a un diamètre de 1,5 mm et ladite ouverture de ladite chambre tubulaire (14) a un diamètre de 13 mm.

5. Appareil d'émission de vapeur de stérilisation selon la revendication 1, dans lequel ledit orifice de la buse (23) a un diamètre de 3 mm et ladite ouverture de ladite chambre tubulaire (14) a un diamètre de 17 mm.

6. Appareil d'émission de vapeur de stérilisation (1) pour l'émission de vapeur de stérilisation, ayant une pression d'émission de la vapeur comprise entre 3 et 10 bars et une température d'émission de la vapeur pouvant atteindre 94 °C afin de stériliser des instruments dentaires, ledit appareil comprenant un corps tubulaire (4) ayant une première (17) et une seconde (11) extrémités de corps tubulaire, ladite première extrémité étant fermée par une paroi d'extrémité (8) incluant une ouverture (9) à laquelle un tuyau d'alimentation en vapeur (2) est raccordé, ladite seconde extrémité (11) dudit corps tubulaire étant ouverte et conçue pour être fermée hermétiquement par un épaulement annulaire (12) d'une partie (13) agencée coaxialement avec ledit corps tubulaire (4), ladite partie (13) ayant une chambre tubulaire (14) qui s'étend axialement et concentriquement par rapport au dit corps tubulaire (4) d'un côté, par rapport au dit épaulement annulaire (12), et un corps cylindrique (15) qui s'étend sur un côté opposé étant logé dans ledit corps tubulaire (4) en formant ainsi une autre chambre (16) entre la première extrémité (17) dudit corps cylindrique (4), et la paroi d'extrémité (8), ledit corps cylindrique (15) présentant une rainure hélicoïdale définissant, avec une surface cylindrique interne dudit corps tubulaire, une conduite hélicoïdale (18) raccordée, d'un côté, à ladite autre chambre (16), dans laquelle ladite vapeur est alimentée, en provenance dudit tuyau d'alimentation en vapeur (2) et, sur l'autre côté, à une rainure annulaire (19) formée dans ledit corps cylindrique (15) et en communication fluidique avec une conduite radiale (20) formée dans ledit corps cylindrique (15) et communicant à son tour avec une buse de conduite (21) formée axialement dans ledit corps cylindrique (15) et étant fermée au niveau d'une extrémité en fond et incluant un orifice de buse (23) communiquant avec ladite chambre tubulaire, ladite chambre tubulaire (14) étant par ailleurs fermée, au niveau du fond de celle-ci, à l'endroit où ledit orifice de buse (23) est formé, et ouverte librement au niveau d'une ouverture opposée de ladite chambre tubulaire, ladite chambre tubulaire étant positionnée autour de la zone dans laquelle l'orifice émerge, ladite chambre tubulaire s'étendant concentriquement avec l'axe longitudinal de la buse sur une section droite prédéterminée grâce à quoi la vapeur émergeant de l'orifice de la buse est poussée à s'écouler à l'intérieur de la chambre tubulaire, ladite chambre tubulaire (14) ayant une section transversale supérieure à une section transversale de l'orifice de la buse (23) d'un facteur compris entre 30 et 150, ledit corps tubulaire (4) incluant en outre un appareil de séchage et de surchauffe de la vapeur à commande de marche-arrêt par une résistance électrique (6), et dans lequel ledit appareil d'émission de vapeur comprend en outre un réservoir à liquide désinfectant ou nettoyant ou aromatique (27) raccordé radialement à ladite partie (13) et communiquant avec ladite chambre tubulaire (14) grâce à un tuyau de vidange de liquide en fond (28), afin d'ajouter à ladite vapeur émise ledit liquide grâce à un effet Venturi provoqué par le flux de vapeur émis.
